(19) Europäisches Patentamt
European Patent Office
Office européen des brevets

(11) Numéro de publication: **0 002 401**
**B1**

(12) **FASCICULE DE BREVET EUROPEEN**

(21) Numéro de dépôt: **78400173.7**

(22) Date de dépôt: **10.11.78**

(51) Int. Cl.³: **C 07 D 295/08,** C 07 D 295/18, C 07 D 295/20, C 07 D 295/22, C 07 D 279/12, A 61 K 31/42, A 61 K 31/425, A 61 K 31/495

(54) **Dérivés de naphtalène, leur préparation et leur application en thérapeutique**

(30) Priorité: **24.11.77 FR 7735307**
**16.10.78 FR 7829413**

(43) Date de publication de la demande:
**13.06.79 Bulletin 79/12**

(45) Mention de la délivrance du brevet:
**10.12.80 Bulletin 80/25**

(84) Etats contractants désignés:
**BE CH DE FR GB LU NL SE**

(56) Documents cités:
**US - A - 2 119 077**

**CHIMICA THERAPEUTICA**
**vol. I, janvier - février 1966**
**J. ANDRIEUX et al.: "Synthèse de nouveaux analogues naphtaléniques de la sérotonine"**
**pages 57—61**

(73) Titulaire: **SYNTHELABO**
**1, avenue de Villars**
**F - 75341 Paris Cedex 07/FR**

(72) Inventeur: **Purcell, Thomas Andrew**
**10, Avenue Gabriel Péri**
**F - 92260 Fontenay aux Roses (FR)**
**Shroot, Braham**
**9bis, rue Condorcet**
**F - 94230 Cachan (FR)**
**Galtier, Daniel Jean Marie**
**20, rue Jean Forest**
**F - 78210 St Cyr l'Ecole (FR)**

(74) Mandataire: **Thouret-Lemaitre, Elisabeth**
**Service Brevets - SYNTHELABO 58, rue de la Glacière**
**F - 75621 Paris Cedex 13 (FR)**

**EP 0 002 401 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Dérivés de naphtalène, leur préparation et leur application en thérapeutique

La présente invention a pour objet de nouveaux dérivés du naphtalène à substitution hétérocycloalkylique en position $\beta$ du noyau naphtalénique ainsi que leurs sels d'addition aux acides pharmaceutiquement acceptables, leur préparation et les médicaments qui en renferment à titre de principe actif.

Les composés de l'invention répondent à la formule I

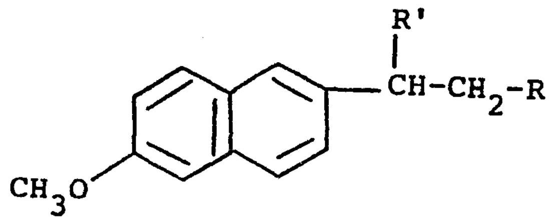

(I)

dans laquelle

R' est un atome d'hydrogène ou le radical méthyle et R représente un radical

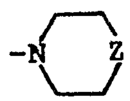

dans lequel Z est un groupe O, S, S → O, $SO_2$ ou $NR_1$ dans lequel $R_1$ est un atome d'hydrogène le radical phényle ou le radical méthyle, $CONR_2R_3$, $COOR_2$, $COR_2$ ou $SO_2R_2$, $R_2$ et $R_3$ représentant chacun, indépendamment l'un de l'autre, un atome d'hydrogène, un radical alkyle de 1 à 4 atomes de carbone, le radical phényle ou le radical $CF_3$.

Les composés de l'invention dans lesquels R' est $CH_3$ présentent un centre asymétrique. Les racémates et les énantiomères font partie de l'invention.

Les composés de l'invention sont utiles en thérapeutique, dans le domaine anti-inflammatoire.

Selon l'invention, on prépare les composés

—soit en faisant réagir l'halogènure d'acide (II)

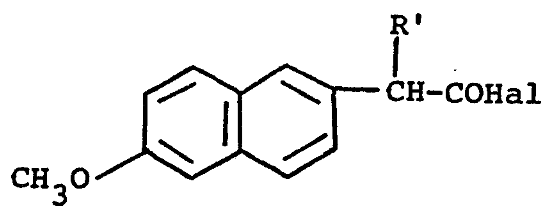

(II)

avec une amine RH (III), dans un solvant tel que le chloroforme à une température de 0 à 25°C, puis en réduisant l'intermédiaire obtenu (IV) dans un solvant tel que le tétrahydrofuranne à une température de 0 à 80°C,

— soit en faisant réagir le mésylate (V)

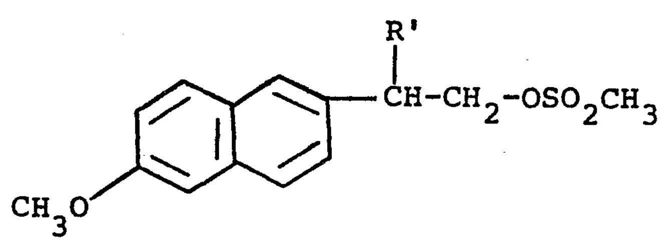

(V)

(obtenu par réaction entre l'alcool naphtalénique correspondant et un halogènure de méthylsulfonyle, dans un solvant tel que $CH_2Cl_2$ à une température de 0 à 25°C), avec une amine RH, dans un solvant tel que l'isopropanol à une température de 25 à 150°C.

Ces deux procédés peuvent subir des variantes selon le radical R, variantes effectuées selon des méthodes classiques.

Les intermédiaires de formule (IV)

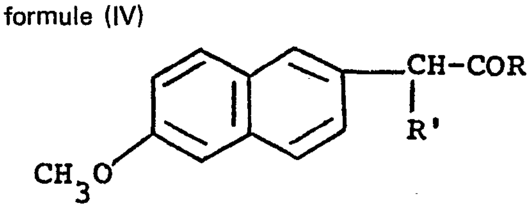

(IV)

sont nouveaux et leur utilisation dans la préparation des composés de l'invention fait partie de l'invention. Certains composés de l'invention peuvent être obtenus, selon des méthodes classiques, à partir d'un dérivé de l'invention.

Ainsi l'oxydation d'un composé I dans lequel R = 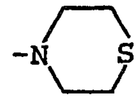

conduit au dérivé I dans lequel R = 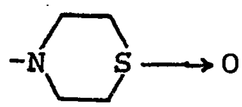

A partir des dérivés I dans lesquels R est 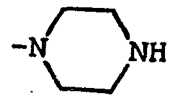

on prépare ceux dans lesquels R est 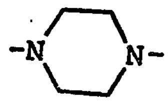

$R_1$ par condensation.

Le composé de formule II et l'acide de départ correspondant sont connus et décrits dans le brevets des Etats Unis n° 4 005 093.

Les composés de l'invention dans lesquels R' est $CH_3$ sont obtenus sous la forme d'énantiomères car le composé de départ II correspondant est lui même optiquement actif.

Les schémas réactionnels ci-après illustrent le préparation des composés.

Schéma A

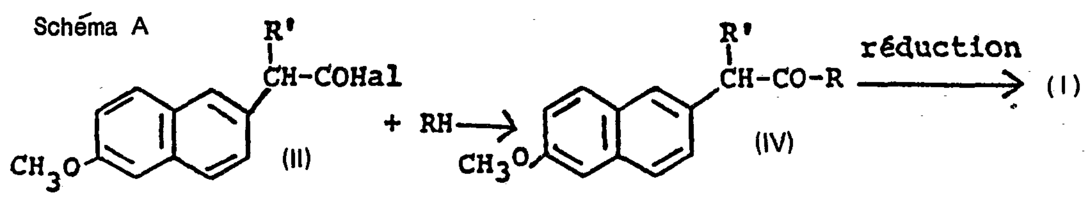


Schéma $A_1$ Variante de A R = 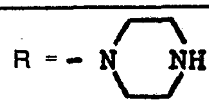

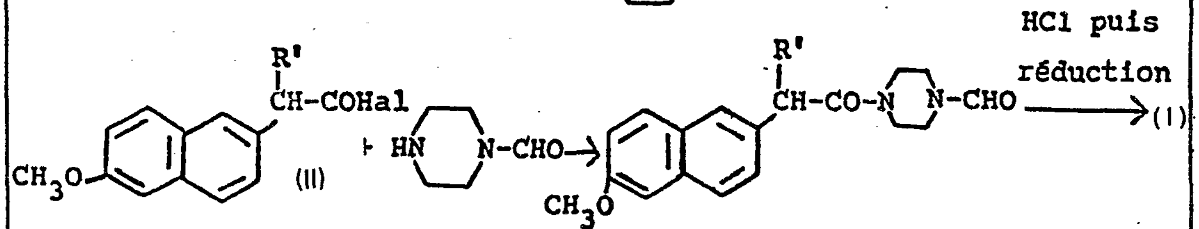


Schéma B

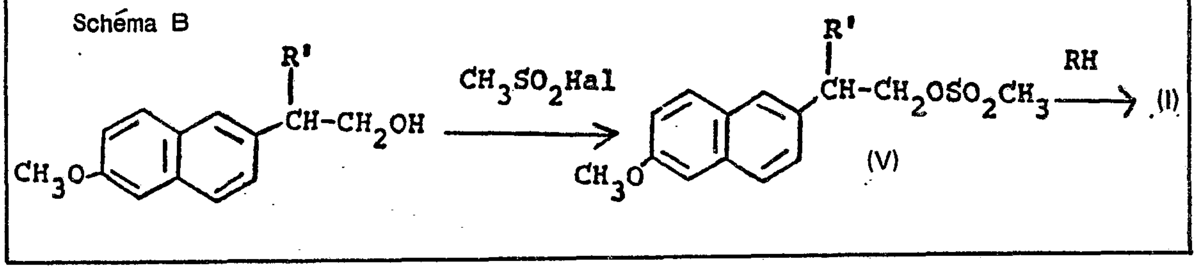

Les exemples suivants illustrent l'invention. Les spectres IR et RMN confirment la structure des composés.

Exemple 1

[(Thiamorpholinyl-4)-2-méthyl-1-éthyl]-2-méthoxy-6-naphtalène

[R′ = $CH_3$ ; R = 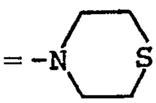 ]

On ajoute du chlorure d'oxalyle (10 ml), goutte à goutte, à une suspension d'acide (méthoxy-6-naphtyl-2)-2 propionique (17,4 g, 75,6 mmoles) dans du benzène (200 ml). Après 2 heures de reflux, le benzène est évaporé sous pression réduite et le résidu est repris dans du dichlorométhane exempt d'alcool (100ml). Cette solution est ajoutée goutte à goutte pendant 30 minutes à une suspension réfrigérée (0°C) de chlorhydrate de thiamorpholine (11,6 g, 83,16 mmoles) et de N-éthylmorpholine (19,12 g, mmoles) dans du dichlorométhane (100 ml). Après une nuit de repos, le milieu réactionnel est lavé successivement à l'eau, à l'acide chlorhydrique (10%), à l'eau, au bicarbonate de sodium aqueux (saturé), à l'eau, puis séché ($Na_2SO_4$) et évaporé. On obtient un solide qui est le composé intermédiaire IV. On ajoute, goutte à goutte, cet amide (5,60 g, 17,78 mmoles) en solution dans du tétrahydrofuranne (50 ml) à une solution agitée de borane (55 mmoles) dans du tétrahydrofuranne (50 ml) et refroidie à 0°C. La solution est ensuite chauffée à la température du reflux durant 2 heures et à nouveau refroidie à 0°C. On ajoute alors de l'eau prudemment pour détruire l'excès de borane et on ajoute de l'acide chlorhydrique dilué.

Après chauffage pendant une heure à la température de reflux, pour détruire les complexes du borane, le tétrahydrofuranne est ensuite distillé sous pression réduite et la suspension aqueuse résultante est mise en partition entre de la soude diluée et de l'acétate d'éthyle.

La phase organique est séparée, lavée à l'eau, séchée ($Na_2SO_4$) et évaporée. On obtient un résidu qui cristallise dans de l'éthanol sous la forme d'un solide blanc. F = 88—89°C.

Exemple 2

[(Oxo-1-thiamorpholinyl-4)-2-méthyl-1-éthyl]-2-méthoxy-6-naphtalène et son chlorhydrate

[R′ = $CH_3$ ; R = 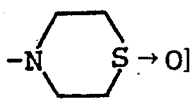 ]

On ajoute goutte à goutte, 0,9 g (0,007 mole) d'eau oxygénée à 30% dans 20 ml d'acide acétique à 2 g (0,0067 mole) de [(thiamorpholinyl-4)-2 méthyl-1-éthyl]-2-méthoxy-6-naphtalène préparé selon la méthode décrite dans l'exemple 1, dissous dans 20 ml d'acide acétique. On abandonne le mélange 5 heures à la température ordinaire, puis on le chauffe 4 heures à la température du reflux. Après refroidissement, on verse le mélange sur de la glace pilée et on l'alcalinise par de l'hydroxyde de sodium 2 N. On extrait la solution basique avec de l'acétate d'éthyle. La phase organique est lavée avec de l'eau, puis une solution de chlorure de sodium, on la sèche sur sulfate de sodium et l'évapore. On recueille 1,2 g d'un solide brun que l'on dissout dans 20 ml d'isopropanol et on ajoute quelques ml d'acide chlorhydrique dans l'éther.

Le chlorhydrate séparé est recristallisé dans de l'isopropanol. On obtient le chlorhydrate d' [(oxo-1-thiamorpholinyl-4)-2-méthyl-1-éthyl]-2-méthoxy-6-naphtalène fondant à 230—231°C.

Exemple 3

[(Dioxo-1, 1-thiamorpholinyl-4)-2-méthyl-1-éthyl]-2-méthoxy-6-naphtalène

[R′ = $CH_3$ ; R = 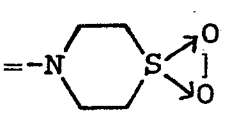

On prépare le méthoxy-6-naphtyl-(2)-$\alpha$-méthyl-acétothiamorpholide (F. 144—146°C) selon la méthode décrite dans l'exemple 1. 22,9 g (0,066 mole) de ce composé sont suspendus dans 250 ml d'acide acétique refroidi au bainmarie. On ajoute goutte à goutte 16,5 g (0,145 mole) d'eau oxygénée à 30%. Quand l'addition est totale, on chauffe 2 heures à la température du reflux. Après refroidissement, on ajoute encore 5 ml d'eau oxygénée à 30% et on poursuit le reflux pendant 2 heures. On laisse le mélange au repos pendant une nuit. On détruit l'excés d'eau oxygénée par addition de sulfate acide de sodium en solution dans l'eau et on évapore la solution presque à siccité. On reprend le résidu dans du sulfate acide de sodium dilué et de l'acétate d'éthyle. On lave la phase organique avec une solution de carbonate de sodium, de l'eau et une solution de chlorure de sodium et on evapore; on recueille un solide brun léger que l'on passe rapidement dans une colonne de gel de silice (200 g), en éluant avec

du chloroforme. On évapore les fractions convenables, les réunit et les cristallise dans l'éthanol pour obtenir 11 g de dioxo-1,1-méthoxy-6-naphtyl-(2)-méthyl-acéto-thiamorpholide sous forme d'un solide blanc fondant à 191—192°C. Cet amide est réduit par $BH_3$/THF selon la description de l'exemple 1.

Le [(dioxo-1, 1-thiamorpholinyl-4)-2-méthyl-1-éthyl]-2-méthoxy-6-naphtalène fond à 108-108,5°C.

Exemple 4

[(Pipérazino-2-méthyl-1-éthyl)]-2-méthoxy-6-naphtalène et son dichlorhydrate

[R′ = $CH_3$ ; R = 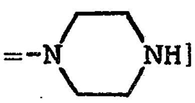]

5,7 g (0,05 mole) de N-formyl-pipérazine sont dissous dans 60 ml de dichlorométhane. On ajoute alors 6,3 ml de N-éthyl-morpholine et on refroidit le mélange à 0°C. A ce mélange, on ajoute goutte à goutte, 12,4 g (0,05 mole) de chlorure de [méthoxy-6-naphtyl-(2)]-2 méthyl-2-acétyle dans 50 ml de dichlorométhane. Après addition totale, on agite le mélange à température ordinaire pendant 30 minutes. On lave la solution avec de l'eau, de l'acide chlorhydrique 1 N du carbonate de sodium à 8% et encore de l'eau, on la sèche sur du sulfate de sodium et on l'évapore. Le résidu huileux est agité avec de l'éther et le mélange est mis en réfrigérateur. L'huile cristallise après 4 jours.

On filtre et sèche et on recueille 11 g de méthoxy-6-naphtyl-(2)-α-méthyl-acéto-(formyl-4 pipérazide) qui fond à 108—109°C et est utilisé aussitôt pour l'étape suivante.

10 g de ce composé brut sont dissous dans 90 ml de méthanol, on ajoute à 0°C, 10 ml d'acide chlorhydrique concentré. On laisse revenir, en agitant, ce mélange, à la température ordinaire et on l'y maintient 30 minutes, puis on le chauffe 5 heures à 40°C.

On évapore les solvants et reprend le résidu dans l'eau. Cette solution aqueuse est lavée avec de l'acétate d'éthyle, alcalinisée par de l'hydroxyde de sodium 4 N et extraite avec de l'acétate d'éthyle. La phase organique est lavée avec de l'eau, séchée sur sulfate de sodium et évaporée. Le résidu huileux cristallise par agitation avec de l'éther. Il fond à 115—116°C.

Le méthoxy-6-naphtyl-(2)-α-méthyl-acéto-pipérazide ainsi obtenu est utilisé immédiatement pour l'étape suivante. On le réduit avec du borane en solution dans du tétrahydrofuranne (THF) (selon la méthode décrite dans l'exemple 1).

Le [pipérazino-2-méthyl-1-éthyl]-2 méthoxy-6-naphtalène qui, purifié par chromatographie sur silice, avec élution par un mélange $CHCl_3/(C_2H_5)_3$ N (98/2), et transformé en dichlorhydrate dans l'isopropanol et l'éther, fond à 265—270°C (déc.) (P.F. du sel).

Exemple 5

[(Méthyl-4-pipérazino)-2-méthyl-1-éthyl]-2-méthoxy-6-naphtalène

[R′ = $CH_3$ ; R = 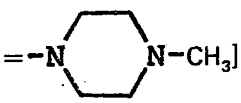]

32,4 g (0,15 mole) de [méthoxy-6-naphtyl-(2)]-2-propanol-1 et 20 ml de N-éthyl-morpholine sont dissous dans 300 ml de chlorure de méthylène dans un ballon entouré d'un bain de glace. On ajoute goutte à goutte, en agitant, 15 ml de chlorure de méthane-sulfonyle dans 30 ml de chlorure de méthylène. On laisse le mélange au repos pendant une nuit. Le solide précipité est dissous par addition de 150 ml de chlorure de méthylène et la solution est lavée avec de l'eau, puis avec une solution de chlorure de sodium et on l'évapore; on recueille 50 g d'un solide qu'on lave avec de l'éther, on obtient le méthanesulfonate de [méthoxy-6-naphtyl-(2)]-2-propyle-2 sous forme d'un solide blanchâtre que l'on utilise sans purification pour la suite de la synthèse. On en a cependant recristallisé une petite portion dans l'acétone pour l'identification (F. 139—141°C).

On chauffe à la température du reflux, 2,94 g (0,01 mole) du composé précédent et 2,00 g (0,02 mole) de N-méthylpipérazine, dans 40 ml d'isopropanol pendant 20 heures. La solution est évaporée et le résidu est repris par l'eau et l'acétate d'éthyle. On sépare la couche organique qu'on lave avec de l'eau jusqu'à pH7, puis avec de l'acide chlorhydrique dilué. L'extrait acid est séparé et lavé avec de l'acétate d'éthyle neuf, alcalinisé par de l'hydroxyde de sodium 4 N et extrait avec de l'acétate d'éthyle. On sépare la phase organique et la lave avec de l'eau, puis une solution de chlorure de sodium; on la sèche sur sulfate de sodium et on l'évapore pour recueillir le [(méthyl-4-pipérazino)-2-méthyl-1-éthyl]-2 méthoxy-6-naphtalène. Après recristallisation dans l'isopropanol le composé fond à 111—112°C.

Exemple 6

[(Ethoxy-carbonyl-4-pipérazino)-2 méthyl-1-éthyl]-2 méthoxy-6-naphtalène et son chlorhydrate

[R' = $CH_3$ ; R = 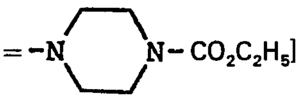

1,4 g (0,0044 mole) de dichlorhydrate de (pipérazino-2-méthyl-1-éthyl)-2-méthoxy-6-naphtalène obtenu dans l'exemple 4 sont dissous dans un mélange de 20 ml de chlorure de méthylène et 1,8 ml (0,014 mole) de N-éthyl-morpholine à 0°C, tout en agitant. Poursuivant l'agitation, on ajoute goutte à goutte, 0,42 ml (0,0044 mole) de chloroformate d'éthyle. Quand l'addition est terminée, on poursuit l'agitation encore 30 minutes en maintenant la température à 0°C. On lave alors le mélange avec de l'eau, on sépare la phase organique et on la sèche sur sulfate de sodium et on l'évapore. L'huile obtenue est reprise dans l'éther et, par addition d'acide chlorhydrique dans l'éther, elle est transformée en son chlorhydrate qui fond à 210—213°C après recristallisation dans l'isopropanol.

Exemple 7

[(Isopropylaminocarbonyl - 4 - pipérazino) - 2 - méthyl - 1 - éthyl] - 2 méthoxy - 6 - naphtalène et son chlorhydrate

[R' = $CH_3$ ; R = 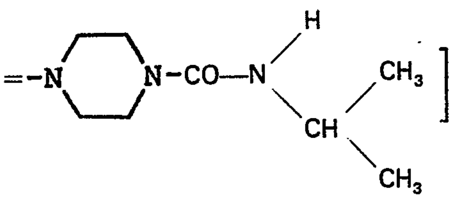

On dissout 15,32 g (0,056 mole) de (pipérazino-2 méthyl-1-éthyl)-2-méthoxy-6-naphtalène dans 300 ml de dichlorométhane et on refroidit la solution à l'aide d'un bain de glace. On ajoute, goutte à goutte, tout en agitant, 6.0 ml (0,061 mole) d'isocyanate d'isopropyle. On laisse revenir le mélange réactionnel à la température ambiante et on le laisse au repos pendant la nuit. On lave ensuite la solution plusieurs fois avec de l'eau, on sèche sur $Na_2SO_4$ et on évapore. On fait cristalliser le résidu dans de l'éther. F = 128—9°C.

On prépare le chlorhydrate du composé en ajoutant de l'éther chlorhydrique à une solution de la base dans de l'éthanol. Le précipité obtenu est recristallisé dans de l'éthanol. F = 234—5°C.

Exemple 8

[(Benzoyl-4-pipérazino)-2 méthyl-1-éthyl]-2-méthoxy-6-naphtalène

[R' = $CH_3$ ; R = 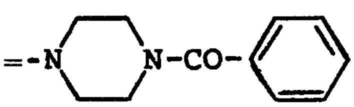 ]

A une solution de (pipérazino-2-méthyl-1-éthyl)-2-méthoxy-6-naphtalène (1 42 g, 5 mmoles) et de N-éthylmorpholine (0,65 ml, 5 mmoles) dans 15 ml de dichlorométhane, on ajoute à 0°C du chlorure de benzoyle (0,58 ml, 5 mmoles). On laisse revenir à la température ambiante et on agite 3 heures. On lave le milieu réactionnel avec de l'eau et on sèche sur sulfate de sodium. Par concentration on obtient un résidu huileux, qui se dissout dans de l'éther et cristallise. On recristallise le composé dans de l'acétate d'éthyle (15 ml).

F = 97°C.

Dans le tableau I suivant sont représentès les composés de l'invention préparés à titre d'exemples.

TABLEAU I

| Composé n° | R' | R | Point de fusion (°C) |
|---|---|---|---|
| 1 | H | 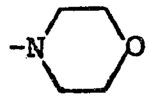 | base 115–117 |
| 2 | $CH_3$ | 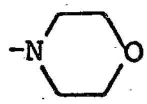 | base 118–120 |
| 3 (ex 1) | $CH_3$ | 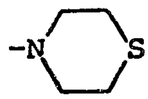 | base 88–89 |
| 4 (ex 2) | $CH_3$ | 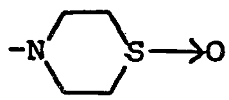 | chlorhydrate 230–231 |
| 5 (ex 3) | $CH_3$ | 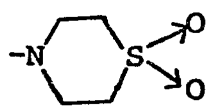 | base 108–108,5 |
| 6 (ex 6) | $CH_3$ | 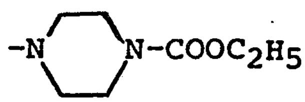 | chlorhydrate 210–213 |
| 7 | $CH_3$ | 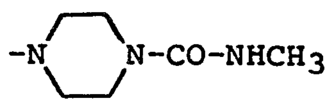 | base 127 |
| 8 (ex 4) | $CH_3$ | 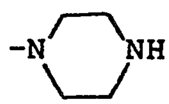 | dichlorhydrate 265–270 (déc) |
| 9 (ex 5) | $CH_3$ | 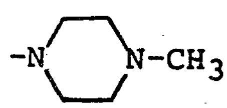 | base 111–112 |
| 10 | $CH_3$ | 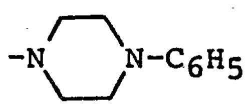 | base 187–189 |
| 11 | $CH_3$ | 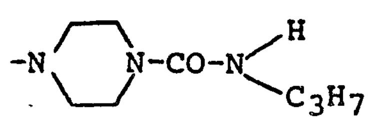 | 144 |

TABLEAU I (fin)

| Composé n° | R' | R | Point de fusion (°C) |
|---|---|---|---|
| 12 (ex 7) | $CH_3$ | 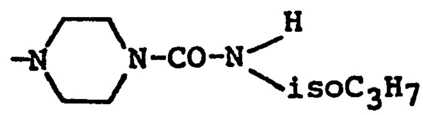 | 129 |
| 13 | $CH_3$ | 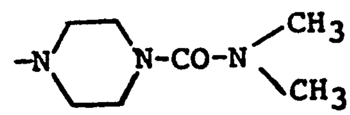 | 114–115 |
| 14 | $CH_3$ | 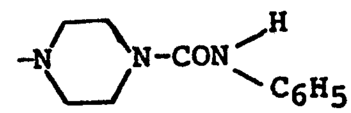 | 152 |
| 15 | $CH_3$ | 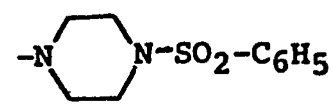 | 192 |
| 16 | $CH_3$ | 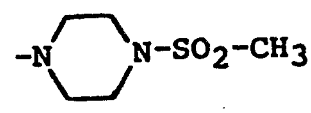 | 153 |
| 17 | $CH_3$ | 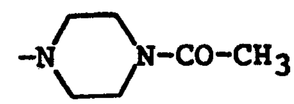 | 141 |
| 18 (ex 8) | $CH_3$ | 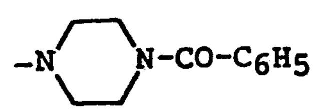 | 97 |
| 19 | $CH_3$ | 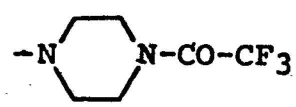 | 93 |

Les composés de l'invention ont été soumis à une série d'essais pharmacologiques qui ont mis en évidence leur activité analgésique, anti-inflammatoire et/ou antipyrétique.

*Toxicité aiguë* Les essais ont porté sur des souris des deux sexes, de souche CD 1, d'un poids moyen de 20 g.

Les doses létales 50% ont été calculées graphiquement.

La majorité des composés de l'invention sont peu toxiques, leur DL 50 est en général supérieure à 1000 mg/kg, par voie orale.

*Activité antiinflammatoire* Cette activité a été déterminée par le test de l'oedème de la patte provoqué chez le Rat Sherman par la carragénine, selon la technique de Winter et coll. (Proc. Soc. Exp. Biol. Med. 1962, *14,* 544). On détermine la dose active 40%, (DE 40) pour les composés. Elle est d'environ 15 à 200 mg/kg par voie orale.

Les composés de l'invention sont utilisables en thérapeutique humaine et vétérinaire, notamment dans le traitement des états inflammatoires, tels que l'arthrite.

La voie d'administration peut être orale, endorectale ou parentérale.

Les composés peuvent être présentés sous toute forme pharmaceutique appropriée pour la voie orale, parentérale ou endorectale telle que comprimés, capsules, gelules, suppositoires, solutions

buvables ou injectables, suspensions, pommades, etc., seuls ou en association avec tout excipient approprié. La posologie quotidienne peut aller de 200 mg à 4000 mg, en une ou plusieurs doses.

**Revendications**

1. Dérivés du naphtalène répondant à la formulae I

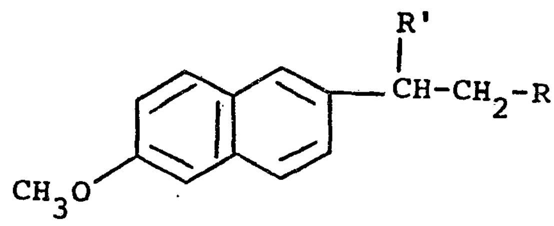

(I)

dans laquelle
R′ est un atome d'hydrogène ou le radical méthyle et R représente un radical

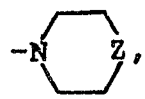

dans lequel Z est un groupe O, S, S → O, $SO_2$ ou $NR_1$ dans lequel $R_1$ est un atome d'hydrogène le radical phényle ou le radical méthyle, $CONR_2R_3$, $COOR_2$, $COR_2$ ou $SO_2R_2$, $R_2$ et $R_3$ représentant chacun, indépendamment l'un de l'autre, un atome d'hydrogène, un radical alkyle de 1 à 4 atomes de carbone, le radical phényle ou le radical $CF_3$, sous la forme de racémates ou d'énantiomères lorsque R′ est $CH_3$, ainsi que leurs sels d'addition aux acides pharmaceutiquement acceptables.

2. Dérivés selon la revendication 1, caractérisés par le fait que R′ est $CH_3$.

3. Le [(dioxo - 1,1 - thiamorpholinyl - 4) - méthyl - 1 - éthyl] - 2 - méthoxy - 6 - naphtalène selon la revendication 1.

4. Le [(isopropylaminocarbonyl - 4 - pipérazino) - 2 - méthyl - 1 - éthyl] - 2 - méthoxy - 6 - naphtalène et son chlorhydrate selon la revendication 1.

5. Procédé de préparation des composés selon la revendication 1, procédé caractérisè en ce qu'on fait réagir l'halogénure d'acide (II)

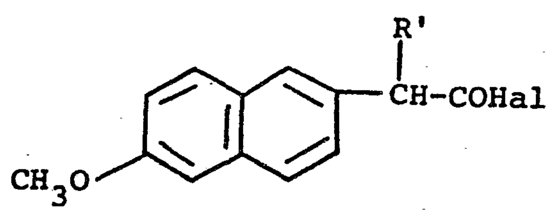

(II)

avec une amine RH (III), puis on réduit l'intermédiaire de formule (IV)

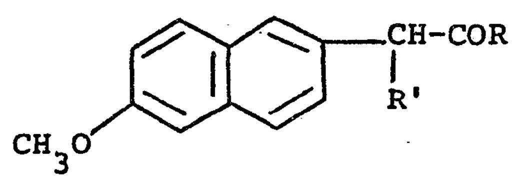

(IV)

ou on fait réagir le mésylate (V)

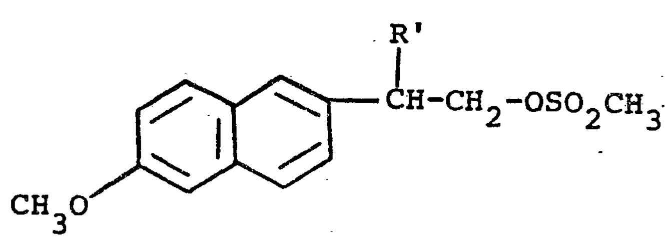

(V)

obtenu par réaction entre l'alcool naphtalénique correspondant et un halogénure de méthylsulfonyle, avec une amine RH (III),

R et R′ ayant les significations de la revendication 1.

6. Médicament caractérisé en ce qu'il contient un composé tel que spécifié dans l'une quelconque des revendications 1 à 4.

**Claims**

1. Naphthalene derivatives corresponding to the formula (I)

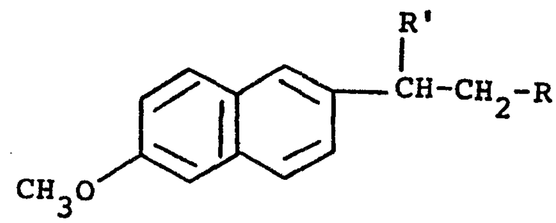

(I)

in which R′ is a hydrogen atom or a methyl radical, and R is a radical

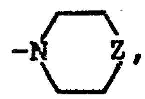

in which Z is O,S,S → O, $SO_2$ or $NR_1$, in which $R_1$ is a hydrogen atom, a phenyl radical, a methyl radical or a $CONR_2R_3$, $COOR_2$, $COR_2$ or $SO_2R_2$ radical, $R_2$ and $R_3$ each representing, independently of one another, a hydrogen atom, a $C_{1-4}$alkyl radical, a phenyl radical or a $CF_3$ radical, in the form of racemates or enantiomers when R′ is $CH_3$, and also, where appropriate, their addition salts with pharmaceutically acceptable acids.

2. Derivatives according to claim 1, wherein R′ is $CH_3$.

3. The 2 - [2 - (1,1 - dioxo - thiamorpholin - 4 - yl) - 1 - methyl - ethyl] - 6 - methoxy - naphthalene, according to claim 1.

4. The 2 - [2 - (4 - isopropylaminocarbonyl - piperazino) - 1 - methylethyl] - 6 - methoxy - naphthalene and its hydrochloride, according to claim 1.

5. A process for preparing compounds according to claim 1, in which process

—an acid halide of formula (II)

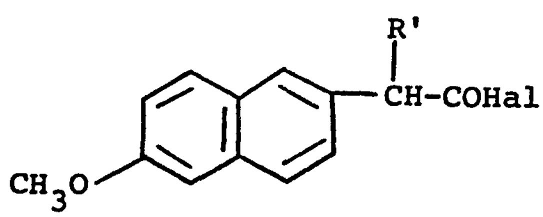

(II)

is reacted with the amine RH (III), and the resulting intermediate of the formula (IV)

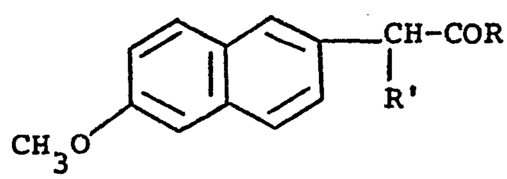

(IV)

is then reduced

—or a mesylate of the formula (V)

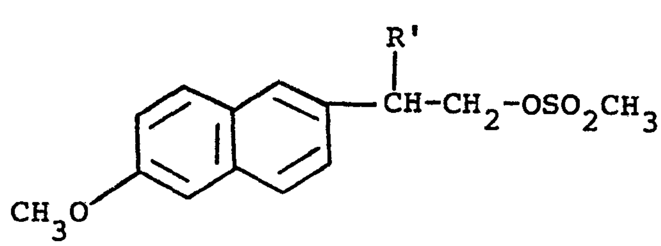

(V)

obtained by reacting the corresponding naphthalenic alcohol with a methylsulphonyl halide, is reacted with the amine RH (III), R and R′ having the meanings given in claim 1.

6. A pharmaceutical composition characterized in that it contains a compound according to any one of claims 1 to 4.

**Patentansprüche**

1. Naphthalinderivate der allgemeinen Formel (I)

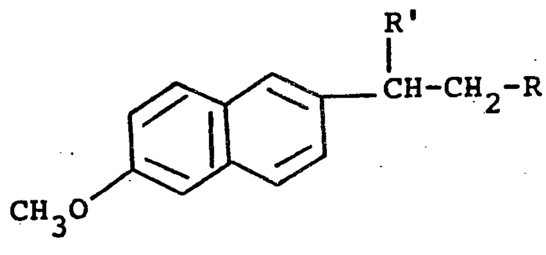

(I)

in der bedeuten:
R′ Wasserstoff oder Methyl und R eine Gruppe

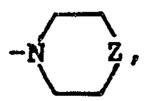,

in der Z für O,S,S → O, $SO_2$ oder $NR_1$ steht, wobei $R_1$ Wasserstoff, Phenyl, Methyl, $CONR_2R_3$, $COOR_2$ oder $SO_2R_2$ bedeutet, und $R_2$ und $R_3$ unabhängig voneinander Wasserstoff, Alkyl mit 1—4 Kohlenstoffatomen, Phenyl oder $CF_3$ darstellen, in Form von Racematen oder Enantiomeren, wenn R′ für Methyl steht, sowie deren pharmazeutisch annehmbaren Säureadditionssalze.

2. Verbindungen nach Anspruch 1, dadurch gekennzeichnet, daß R′ $CH_3$ ist.

3. 2 - [2 - (1,1 - dioxo - thiamorpholin - 4 - yl) - 1 - methyl - äthyl] - 6 - methoxynaphthalin, nach Anspruch 1.

4. 2 - [2 - (4 - isopropylaminocarbonyl - piperazino) - 1 - methyl - äthyl] - 6 - methoxy - naphthalin und dessen Chlorhydrat, nach Anspruch 1.

5. Verfahren zur Herstellung von Verbindungen nach Anspruch 1, dadurch gekennzeichnet, daß man entweder ein Säurehalogenid der allgemeinen Formel

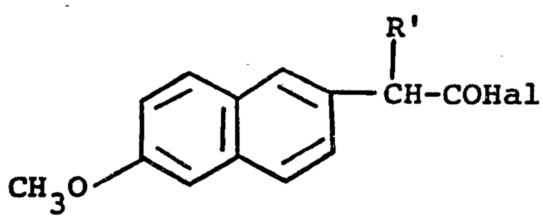

(II)

mit einem Amin der allgemeinen Formel RH (III), reagieren läßt, wonach man die so erhaltene Verbindung der allgemeinen Formel

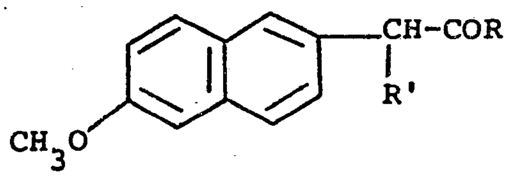

(IV)

reduziert,
oder ein durch Umsetzung eines entsprechenden Naphthylalkohols mit einem Methylsulfonylhalogenid erhaltenes Mesylat der allgemeinen Formel

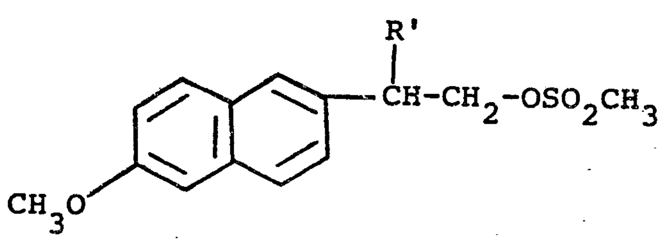

(V)

mit einem Amin der allgemeinen Formel RH (III) reagieren läßt wobei in den Formeln R und R' obige Bedeutungen haben.

6. Arzneimittel, dadurch gekennzeichnet, daß sie eine Verbindung gemäß den Ansprüchen 1 bis 4 enthält.